# EUROPEAN PATENT SPECIFICATION

(11) **EP 4 259 706 B1**
(45) Date of publication and mention of the grant of the patent: **29.01.2025**
(21) Application number: 21836231.7
(22) Date of filing: 06.12.2021
(51) Int. Cl.: C08J 11/10, B09B 3/60

(54) **PLASTIC-DEGRADING SOLUTION**
KUNSTSTOFFABBAUENDE LÖSUNG
SOLUTION POUR DÉGRADATION DE MATIÈRE PLASTIQUE

(30) Priority: 09.12.2020 IT 202000030200
(43) Date of publication of application: 18.10.2023
(73) Proprietor: Innovazione e Sviluppo Sostenibile S.r.l., 20123 Torino (IT)
(72) Inventor: BUONAMICI, Guglielmo, 56017 San Giuliano Terme (IT)
(74) Representative: Lunati & Mazzoni S.r.L.
(86) International application number: PCT/IB2021/061353
(87) International publication number: WO 2022/123421

(56) References cited:
- WO-A1-2015/173265
- WO-A1-2015/173265
- CN-A- 104 232 501
- CN-A- 104 232 501
- CN-A- 108 588 052
- CN-A- 108 588 052
- CN-A- 109 467 742
- CN-A- 109 467 742
- CN-A- 109 929 789
- CN-A- 109 929 789
- CN-A- 109 957 140
- CN-A- 109 957 140
- JP-A- H 114 680
- JP-A- H 114 680
- JP-A- H08 140 666
- JP-A- H08 140 666

## Description

The present invention relates to a plastic-degrading solution**.**

In particular, the invention relates to a mixture of more substances (in liquid or gaseous or preferably solid phase) suitably selected and mixed so as to have a compound configured to degrade plastic materials.

To date, the most common plastics on the market are mainly polyethylene (PE) used for bags, bottles, toys, and packaging; polypropylene (PP) used for furniture items, food containers; polyvinyl chloride (PVC) used for trays, films, pipes; polyethylene terephthalate (PET) used for synthetic fibres, bottles, tapes; and polystyrene (PS) used for food trays, cutlery, plates, glasses.

The main techniques for the disposal of plastic include an initial selection and treatment; a separation from impurities, and therefore a subdivision by type of polymer and therefore subjected to a specific treatment. For example, in the case of PET and PE the recycling process can be mechanical with the obtaining of granules to be used to obtain new objects; or chemical in which there is a plastic-degrading solution which, through a process called depolymerization, breaks up the basic molecules of the plastic (polymers) and obtain the starting raw materials (monomers).

One possible plastic-degrading solution includes bacteria capable of digesting certain nylon manufacturing by-products known as nylonase.

Other plastic-degrading solutions involve the adoption of enzymes which, through a thermomechanical process called hydrolase, degrade plastic materials. Examples of such enzymes are the PETase enzyme, the MHETase enzyme.

The known technique described includes some important drawbacks.

In particular, mechanical recycling allows for the recycling of almost all plastics, but is severely limited by the fact that it allows a limited number of recycles and therefore a reduced number of re-uses of the plastic.

Mechanical recycling, while allowing a greater number of recycles, has many drawbacks. In fact, the reagents used to break the bonds allow the recycling of only part of the plastic materials and, above all, they are slow.

A not secondary drawback of chemical recycling is represented by the fact that the production methods, being the plastic-degrading solutions adopted mostly of chemical origin, are particularly expensive and relatively polluting.

Therefore, chemical recycling, although in future projections a preferable solution, has much higher plastic recycling costs and times than mechanical recycling which is currently preferable.

In this situation, the technical task underlying the present invention is to devise a plastic-degrading solution capable of substantially obviating at least part of the aforementioned drawbacks.

Plastic-degrading solutions based on fungi or extracts of natural origin are known. Examples of plastic-degrading solution are disclosed in CN 108 588 052 A, CN 109 467 742 A, JP H08 140666 A, WO 2015/173265 A1, JP H11 4680 A, CN 109 957 140 A, CN 109 929 789 A and CN 104 232 501 B.

Within the scope of said technical task, an important object of the invention is to obtain a plastic-degrading solution which allows chemical recycling of any inexpensive and relatively fast plastic.

The present invention discloses a plastic-degrading solution characterized by comprising Flavobacterium, Enterobacter asburiae, Ideonella sakaiensis, Sphingomonas, Bacillus subtilis and extracts of Fusarium oxysporum, Purpureocillium lilacinum, Pestalotiopsis microspora, Trichoderma arzianum and Alcaligenes eutrophus.

In particular aspect of the present invention, the plastic-degrading solution further comprises titanium dioxide.

In a further aspect of the present invention, the plastic-degrading solution further comprises at least one of Thermobifida fusca, Bacillus megaterium, Pseudomonas aeruginosa secondary and Bacillus sp. Secondary YP1.

In another aspect of the present invention, the plastic-degrading solution further comprises extracts of at least one of Aspergillus tubingensis and Saccharomyces cerevisae.

In the present disclosure, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

The measurements and data reported in this text are to be considered, unless otherwise indicated, as carried out in the ICAO International Standard Atmosphere (ISO 2533).

The plastic-degrading solution according to the invention is configured to be used in a plastic-degrading process. The solution and therefore the process are configured to allow the environment to be reclaimed from plastics or their derivatives such as polyethylene substances, hydrocarbons, polyurethanes, PET.

In particular, it is configured to degrade plastic material. Said plastic material can comprise one or more of polyethylene (in the various forms), polyurethane, polypropylene, polyvinyl chloride and polystyrene.

Examples of a plastic-degrading solution can be a mixture of one or more extracts (in liquid or gaseous or preferably solid phase) having an almost exclusive natural origin and in detail vegetable with the opportune exception for titanium dioxide (or other substance with the same function) as described below. An example of plastic-degrading solution can include fungi suitably selected from Fusarium oxysporum, Purpureocillium lilacinum, Pestalotiopsis microspora, Aspergillus tubingensis, Trichoderma arzianum and Saccharomyces cerevisae.

An example of plastic-degrading solution can comprise extracts of Purpureocillium lilacinum suitably deriving from root systems of Purpureocillium lilacinum. Purpureocillium lilacinum extracts may comprise serine proteases.

An example of plastic-degrading solution can comprise extracts of Fusarium oxysporum suitably deriving from host plants parasitized by Fusarium oxysporum including Medicago sativa and Solanum lycopersicum and Musa acuminata.

An example of plastic-degrading solution can comprise extracts of Purpureocillium lilacinum suitably deriving from uncultivated soils. Purpureocillium lilacinum extracts may comprise serine proteases.

An example of plastic-degrading solution can comprise extracts of Pestalotiopsis microspora suitably deriving from hosts represented by arboreal plants such as Cupressus sempervirens and Thuia occidentalis.

Pestalotiopsis microspora extracts may comprise serine hydrolase.

In addition, the extracts of Pestalotiopsis microspora may comprise one or more and in detail the entirety of torreyanic acid and dimeric quinone.

An example of plastic-degrading solution can comprise extracts of Trichoderma arzianum suitably deriving from uncultivated soils.

Trichoderma arzianum extracts may comprise chitinases.

In addition, the extracts of Trichoderma arzianum can comprise one or more and in detail the totality of cellulases and hemicellulases.

An example of plastic-degrading solution can include extracts of Aspergillus tubingensis suitably deriving from uncultivated and cultivated soils and on various fruit plants and in pathological manifestations with the formation of molds of the Pome fruit. The extracts of Aspergillus tubingensis can comprise one or more and in detail the totality of amylase, lipase, glucose oxidase, phytase, xylanase, acid phosphatase and xilosidas.

An example of plastic-degrading solution can comprise extracts of Saccharomyces cerevisae suitably deriving from residues and waste from winemaking and beer production and other fermentation processes.

Saccharomyces cerevisae extracts may include invertase.

The mycetes described above and extracted in detail are preferably obtainable from cultural substrates suitably enriched with suitable nutrients such as e.g., nitrogen, molasses, ammonia.

In an example the plastic-degrading solution can include bacteria suitably selected from among Flavobacterium, Enterobacter asburiae, Ideonella sakaiensis, Thermobifida fusca, Sphingomonas, Bacillu ssubtilis, Bacillus megaterium, Pseudomonas aeruginosa, putida and Bacillus sp. yp1.

In detail, in an example the plastic-degrading solution can comprise bacteria of the Flavobacterium type.

In an example the plastic-degrading solution may comprise Enterobacter asburiae type bacteria.

In an example the plastic-degrading solution may comprise bacteria of the Ideonella sakaiensis type.

In an example the plastic-degrading solution can comprise bacteria of the Sphingomonas type preferably belonging to the Sphingomonas sp. 2MPII.

In an example the plastic-degrading solution may comprise bacteria of the Bacillus subtilis type.

In an example the plastic-degrading solution can comprise bacteria of the thermobifidafusca type.

In an example the plastic-degrading solution may comprise bacteria of the Bacillus megaterium type.

In an example the plastic-degrading solution can comprise bacteria of the Pseudomonas aeruginosa and/or putica type.

In an example the plastic-degrading solution can comprise bacteria of the Bacillus sp YP1. type.

In an example the plastic-degrading solution may comprise bacteria of the Alcaligenes eutrophus type.

In an example the plastic-degrading solution may comprise feeding bacteria, i.e., bacterial generics for the specific purposes of providing nourishment to the microbial population of the formulation.

The feeding bacteria can comprise one or more and in detail all Brevibacterium, Microbacterium and Corynebacterium

Furthermore, in an example the plastic-degrading solution can comprise sugar such as cane molasses or beet, hydrolyzed starch from corn tubers or Magnoca or Tapioca. Along with sugar.

In an example the plastic-degrading solution may further comprise nitrogen sources such as ammonia and/or ammonium salts.

Herein, it is disclosed also a new plastic-degrading process.

The plastic-degrading process is configured to be carried out using the plastic-degrading solution described above.

The plastic-degrading process can comprise a degradation step in which the plastic material, mixed with the plastic-degrading solution, is decomposed in order to obtain gaseous, solid and/or liquid products.

These products can be used for the production of heat, new objects and/or electricity.

In the degradation phase the plastic material can be brought to a degradation temperature. The degradation temperature can be substantially between 10°C and 100°C in detail between 20° and 50° and to be precise between 25°C and 35°C. It can be substantially equal to 30°C.

The degradation step can be carried out in a bioreactor.

The degradation phase can be carried out by exposing the plastic material to sunlight.

In the degradation step, the plastic material can be mixed with the plastic-degrading solution which performs a biodegrading action of said material.

In detail, F lavobacterium, thanks to its nylonase enzyme content, acts on the carbon-nitrogen bonds with degradation of the polymers of the plastic material.

In detail, the bacterium Enterobacter asburiae has a particular application in the degradation of polyethylene included in the plastic material. The degration action of polyethylene is assisted by Bacillus sp. YP1 and from Sakaiensis which intervenes in the degradation of polyethylene by hydrolyzing the polymer chains which it uses as a primary source of carbon for nutritional purposes.

The degradative action of the plastic material occurs through the Sakaiensis bacterium which lives and feeds on PET (polyethylene terephthalate). In fact, it identifies as the only source of carbon (fundamental to the bacterium) in plastic polymers where with the help of PETase (produced by Ideonella sakaiensis) it catalyzes the hydrolysis of polyethylene terephthalate.

An important degradation action is defined by the presence of Ideonella sakaiensis which, in collaboration with the PETase enzymes intervening by splitting the plastic material into the intermediates MHET (mono- (2-hydroxyethyl) terephthalic acid) and BHET (bis- (2-hydroxyethyl) terephthalic acid)) and METase (derived from the same Ideonella) (intervening by hydrolyzing the ester bond of MHET forming terephthalic acid and ethylene glycol).

The degradative action of the plastic material and in particular of the polyethylene described above can be increased by the presence of Purpureocillium lilacinum which, thanks to the particular supply of serine proteases and other enzymes, carries out a significant biological activity in the destruction of the polyethylene purposely made more bioactive by a greater production of metabolic energy in the form of ATP expressed by the same presence of the serine of Purpureocillium lilacinum.

To this we can add a significant action of the Enterobacter bacterium in which there is an important degradation action of polyethylene substances (one of the main components of the plastic material). This action is synergistically assisted by the Sphingomonas capable of degrading the polyethylene materials

Said degrading action of the plastic material and in detail of the polyethylene is integrated by the Purpureocillium which, thanks to its supply of important enzymes including the serine protease, carries out a significant biological activity in the destruction of the polyethylene purposely made more bioactive by a greater production of metabolic energy in the form of ATP expressed by the same presence of the serine of Purpureocillium.

The described reaction takes place through the dehydration carried out by the enzyme serine dehydratase by removing a molecule of water in the form of a hydroxyl group and finally subtracting a hydrogen atom from the carbon.

Furthermore, the presence of Sphingomonas guarantees to the plastic-degrading solution the possibility of both degrading the hydrophobic polycyclic aromatic hydrocarbons and an important degradation action of 2-methyl phenanthrene.

To the aforementioned degrading actions of the plastic material are added those of pseudomonas aeruginosa capable of attacking the polymeric chemical bonds of polyurethane plastics and of Pestalotiopsis microspora, which in collaboration with Pseudomonas aeruginosa, exploits its own supply of serine hydrolase to metabolize polyurethane.

It is highlighted that the aforementioned actions are favoured by the inclusion of Bacillus subtilis which, thanks to its content of biosurfactant (surfactin), favours the accessibility of microorganisms and therefore allows for greater biodegradation in a short time.

The plastic-degrading actions of the solution are accentuated by the presence of Thermobifida fusca (a thermophilic bacterium used in the degradation formulation of plastic materials) and Bacillus megaterium (a Gram-positive bacterium capable of contributing to the degradation of plastic materials).

To the above-described actions of bacteria and fungi is added that defined by Trichoderma arzianum which produces particular plastic-degrading enzymes (including those mentioned above) and in particular cellulase, hemicellulase and above all chitinase (an enzyme capable of degrading aliphatic compounds and plastic materials).

Furthermore, the Alcaligenes eutrophuse interacts with the carbon components of the plastic material defining a strong degradation.

This Aspergillus is able to reproduce enzymes (amylase, lipase, glucose oxidase, phytase, xylanase, acid phosphatase and xylosidase) which, associated with nitrogen compounds, is able to degrade PETs; in particular the amylase produced by A. tubingensis can be used in the production of bioethanol and biofuels and to degrade polyurethane.

Finally, it is highlighted how the presence of titanium dioxide guarantees to the plastic-degrading solution an improved oxidizing and degrading action of the plastic material thanks to its ability to oxidize numerous organic compounds. In particular, titanium dioxide, especially when exposed to light and/or with water and carbon dioxide, catalyses the oxidation of organic residues, i.e. dirt, pollution deposits, plastics and various types of microorganisms).

Furthermore, the production of important enzymes for the action of the plastic-degrading solution is given by Saccharomyces cerevisae which, by inducing fermentation, produces the invertase enzyme which splits sucrose into its specific components, glucose and fructose so as to make them available to microbial organisms. and in detail at least the mycetes of the solution plastic-degrading actions.

To this action is added that of Fusarium oxysporum which, by degrading lignin and waxy substances (polyesters), helps the degradation of plastic by degrading complex carbohydrates.

The plastic-degrading process can comprise a pre-treatment step of the plastic material.

The pre-treatment step can be prior to the degradation step.

The pre-treatment phase includes a first intervention of collection of plastic materials in polluted sites, their placement in storage tanks and their subsequent grinding with a special shredder system.

The pre-treatment step can be performed in a tank suitably comprising an agitator configured to move at least the plastic material in the tank.

The pre-treatment step can be performed by adding the plastic material with a mixture that loosens the chemical bonds of the plastic polymers and thus favouring the degradation described above in the degradation step.

The loosening mixture may comprise suitably salted water (e.g., sea water; and sulfuric acid).

The loosening mixture can be placed in the tub after the plastic material.

The release of the loosening mixture can be at high pressure and in detail at a pressure of at least 2 atm.

The plastic-degrading process can comprise a step of irradiation of the plastic material.

The irradiation phase can be prior to the degradation phase.

The irradiation phase can be subsequent to the pre-treatment phase.

In the irradiation phase, the plastic material can be subjected to radiation (preferably with UV rays) configured to loosen the bonds of the plastic polymers.

The plastic-degrading solution according to the invention achieves important advantages.

In fact, contrary to the known chemical degradation processes of plastic, the plastic-degrading solution and therefore the plastic-degrading process using said plastic-degrading solution allows a relatively rapid degradation of the plastic material. These actions are synergistically carried out by the enzymes contained primarily in Flavobacterium, Enterobacter asburiae, Ideonella sakaiensis, Sphingomonas, Bacillus subtilis and extracts of Fusarium oxysporum, Purpureocillium lilacinum, Pestalotiopsis microspora, Trichoderma arzianum and Alcaligenes eutrophus. In fact, Flavobacterium (being equipped with nylonase enzymes, dimeric hydrolase, 6-aminohexanoic linear hydrolase oligomer and 6-aminohexanoic acid hydrolase derived from Frameshift mutation) Trichoderma arzianum (containing chitinase) and Alcaligenes (with the use of its PHB enzyme - poly (3-hydroxybutyrate) depolymerase) degrade plastics. These actions are facilitated by Sphingomonas which metabolizes the carbon of plastics. Furthermore, the degradation of plastics is coaudivated by Enterobacter esburiae able (especially in conditions of anaerobiosis) degrading the polymeric bonds and by specific actions of Ideonella sakaiensis (with PETase making a first degradation and ultimately MET hydrolase called first degradation), and above all by degradation polyesters (given by Fusarium oxysporium) of polyethylene (given by Purpureocillium lilacinum containing serine protease) and polyurethane (given by Pestalotiopsis microspora containing serine hydrolase enzyme). The aforementioned actions are amplified by Bacillus subtilis which, by producing surfactin, facilitates the accessibility of said principles and therefore their action.

This significant degradation can be accentuated by Aspergillus tubingensis which, containing amylase, lipase, glucose oxidase, phytase, xylanase, acid phosphotase and xylosidase) helps Pestalotiopsis microspora and other principles in the degradation of polyurethane; from Trichoderma arzianum, Thermobifida fusca degrades and/or Pseudomonas aeruginosa (also rich in exoenzymes capable of metabolizing plastic chemical compounds for energy production).

It also highlights the importance of Saccharomyces cerevisae (containing invertase) produces from sucrose nutrients of the principles of the solution such as glucose and fructose.

Through the use of particular extracellular enzymes, and more precisely exoenzymes, they have been shown to be able to metabolize the chemical compounds of plastic as a source of carbon and used for the production of energy. Another advantage can be identified in the fact that while the known technique allows the degradation of only specific plastics, the plastic-degrading solution allows to degrade plastic material of substantially any type.

An important advantage is represented by the fact that the plastic-degrading solution allows to carry out a particularly simple and economical plastic-degrading process. It should be noted that these advantages have been obtained from the plastic-degrading solution which, thanks to the innovative combination of fungi and bacteria (suitably integrated with titanium dioxide), is particularly effective as demonstrated by the studies carried out by the inventor.

## Claims

1. Plastic-degrading solution **characterized by** comprising Flavobacterium, Enterobacter asburiae, Ideonella sakaiensis, Sphingomonas, Bacillus subtilis and extracts of Fusarium oxysporum, Purpureocillium lilacinum, Pestalotiopsis microspora, Trichoderma arzianum and Alcaligenes eutrophus.

2. Solution (1) according to claim 1, further comprising titanium dioxide,

3. Solution (1) according to at least one previous claim, further comprising at least one of Thermobifida fusca, Bacillus megaterium, Pseudomonas aeruginosa secondary and Bacillus sp. Secondary YP1.

4. Solution (1) according to at least one preceding claim, further comprising extracts of at least one of Aspergillus tubingensis and Saccharomyces cerevisae.

## Patentansprüche

1. Kunststoffabbauende Lösung, **dadurch gekennzeichnet, dass** sie Flavobacterium, Enterobacter asburiae, Ideonella sakaiensis, Sphingomonas, Bacillus subtilis und Extrakte von Fusarium oxysporum, Purpureocillium lilacinum, Pestalotiopsis microspora, Trichoderma arzianum und Alcaligenes eutrophus umfasst.

2. Lösung (1) nach Anspruch 1, ferner umfassend Titandioxid.

3. Lösung (1) nach mindestens einem vorhergehenden Anspruch, ferner umfassend mindestens eines von Thermobifida fusca, Bacillus megaterium, sekundäre Pseudomonas aeruginosa sekundäre und Bacillus sp. sekundäre YP1.

4. Lösung (1) nach mindestens einem vorhergehenden Anspruch, ferner umfassend Extrakte von mindestens einem von Aspergillus tubingensis und Saccharomyces cerevisiae.

## Revendications

1. Solution dégradant le plastique, **caractérisée en ce qu'**elle comprend Flavobacterium, Enterobacter asburiae, Ideonella sakaiensis, Sphingomonas, Bacillus subtilis et des extraits de Fusarium oxysporum, Purpureocillium lilacinum, Pestalotiopsis microspora, Trichoderma arzianum et Alcaligenes eutrophus.

2. Solution (1) selon la revendication 1, comprenant en outre du dioxyde de titane.

3. Solution (1) selon au moins une revendication précédente, comprenant en outre au moins l'un de Thermobifida fusca, Bacillus megaterium, Pseudomonas aeruginosa secondaire et Bacillus sp. secondaire YP1.

4. Solution (1) selon au moins une revendication précédente, comprenant en outre des extraits d'au moins l'un de Aspergillus tubingensis et Saccharomyces cerevisiae.
